# EUROPEAN PATENT SPECIFICATION

(11) **EP 1 292 701 B1**
(45) Date of publication and mention of the grant of the patent: **09.08.2006**
(21) Application number: 01938911.3
(22) Date of filing: 05.06.2001
(51) Int. Cl.: C12Q 1/48, G01N 33/53

(54) **ASSAY FOR DETECTION OF TRANSFERASE ENZYME ACTIVITY IN DRUG SCREENING**
ASSAY ZUR DETEKTION DER TRANSFERASEENZYMAKTIVITÄT BEIM SCREENING VON MEDIKAMENTEN
TEST DE DETECTION DE L'ACTIVITE ENZYMATIQUE DE LA TRANSFERASE DANS LE DEPISTAGE DES DROGUES

(30) Priority: 08.06.2000 IN MA043900
(43) Date of publication of application: 19.03.2003
(73) Proprietor: AstraZeneca AB, 151 85 Södertälje (SE)
(72) Inventor: DESOUSA, Sunita Maria, Bangalore 560003 (IN); SOLAPURE, Suresh, Bangalore 560003 (IN)
(74) Representative: Phillips, Neil Godfrey Alasdair
(86) International application number: PCT/SE2001/001271
(87) International publication number: WO 2001/094622

(56) References cited:
- WO-A1-00/10587
- WO-A1-96/15258
- WO-A1-96/16082
- WO-A1-99/38958
- WO-A1-99/60155
- ARTHUR A. BRANSTROM ET AL.: 'In situ assay for identifying inhibitors of bacterial transglycosylase' FEMS MICROBIOLOGY LETTERS vol. 191, 2000, pages 187 - 190, XP002948888
- DATABASE BIOSIS [Online] BRANSTROM ARTHUR A. ET AL.: 'Assay for identification of inhibitors for bacterial MraY translocase of MurG transferase', XP002948803 Database accession no. PREV200000372168 & ANALYTICAL BIOCHEMISTRY vol. 280, no. 2, 01 May 2000, pages 315 - 319

## Description

The present invention relates to a method for assaying enzymes involved in peptidoglycan biosynthesis in bacteria.

Peptidoglycan is a major component of the bacterial cell wall that gives the wall its shape and strength. It is unique to bacteria and is found in all bacteria, both gram-positive and gram-negative. Peptidoglycan is a polymer of glycan strands that are cross-linked through short peptide bridges. It consists of alternating β1-4 linked residues of *N*-acetyl glucosamine (Glc*N*Ac) and *N*-acetyl muramic acid (Mur*N*Ac). A pentapeptide chain is attached to Mur*N*Ac (Mur*N*Ac-pentapeptide) and cross-linking occurs between these peptide chains.

Biosynthesis of peptidoglycan can be divided into three stages: firstly, synthesis of the precursors in the cytoplasm, secondly, transfer of the precursors to a lipid carrier molecule and, thirdly, insertion of the precursors into the cell wall and coupling to existing peptidoglycan.

The precursors synthesised in the cytoplasm are the sugar nucleotides:
UDP-*N*-acetyl-glucosamine (UDP-Glc*N*Ac) and UDP-*N*-acetylmuramylpentapeptide
(UDP-Mur*N*Ac-pentapeptide).

The second stage, which occurs in the cytoplasmic membrane, is catalysed by two enzymes and involves synthesis of a disaccharide unit on a lipid carrier, undecaprenyl phosphate. The lipid carrier is also involved in the synthesis of other components of the bacterial cell wall.

The first enzyme catalyses the transfer of phosphoryl-*N*-acetyl muramyl pentapeptide from UDP-Mur*N*Ac-pentapeptide to undecaprenol phosphate with the simultaneous release of UMP. This enzyme is called phospho-*N*-acetylmuramyl-pentapeptide translocase (hereafter referred to as "the translocase") and is the product of the gene mra Y in *Escherichia coli.* The product, undecaprenol-pyrophosphate-*N-*acetylmuramylpentapeptide (Lipid-P-P-Mur*N*Ac-pentapeptide) or Lipid I or Lipid linked precursor I is the substrate for the second enzyme.

*N*-acetylglucosaminyl transferase, transfers N-acetylglucosamine from UDP-GlcNAc (with simultaneous release of UDP) to form undecaprenol-pyrophosphoryl-*N-*acetylmuramylpentapeptide-*N*-acetylglucosamine or Lipid II or Lipid linked precursor II. This enzyme is also called UDP-*N*-acetylglucosamine: *N*-acetylmuramyl(pentapeptide)-P-P-undecaprenol-N-acetylglucosamine transferase (hereafter referred to as "the transferase"). The enzyme is the product of the gene murG in *Escherichia coli.*

The translocase and the transferase enzymes are essential for bacterial viability (see respectively D.S. Boyle and W. D. Donachie, *J. Bacteriol*., (1998), **180,** 6429-6432 and D. Mengin-Lecreulx, L. Texier, M. Rousseaue and Y. Van Heijernoot, *J. Bacteriol*., (1991), **173,** 4625-4636).

In the third stage, at the exterior of the cytoplasmic membrane, polymerisation of the glycan occurs. The disaccharide-pentapeptide unit is transferred from the lipid carrier to an existing disaccharide unit or polymer by a peptidoglycan transglycosylase (also referred to as a peptidoglycan polymerase) (hereafter referred to as "the transglycosylase"). The joining of the peptide bridge is catalyzed by peptidoglycan transpeptidase (hereafter referred to as "the transpeptidase"). Both enzyme activities which are essential reside in the same molecule, the penicillin binding proteins (or PBPs), as in PBP 1a or 1b in *Escherichia coli.* These are the products of the ponA and ponB genes respectively, in *Escherichia coli.*

There are several PBPs in the bacterial cell and these can be divided into two classes, the low molecular mass (LMM) and high molecular mass (HMM) PBPs. The HMM PBPs are bifunctional enzymes having both transpeptidase and transglycosylase activity. Of these PBP2 and PBP3 and either PBP1A or PBP1B of *E. coli* have been shown to be essential for cell viability. The LMM PBPs appear to be important but not essential for cell growth (e.g. PBPs 4, 5, 6 *of E. coli* can be deleted resulting in growth defects but the cell survives, see S.A. Denome, P.K. Elf, T.A. Henderson, D.E. Nelson and K.D. Young, *J. Bacteriol*., (1999), **181(13)**, 3981-3993).

On transfer of the disaccharide-pentapeptide unit from the lipid precursor to an existing peptidoglycan chain the lipid is released as a molecule of undecaprenol pyrophosphate. This has to be cleaved by a bacitracin-sensitive undecaprenyl pyrophosphorylase, also called undecaprenol pyrophosphorylase or C55-isoprenyl pyrophosphorylase (hereafter referred to as the "lipid pyrophosphorylase") to generate undecaprenol phosphate which can then re-enter the cycle at the second stage.

Both the translocase and the transferase (mraY and murG gene products, respectively) represent prime targets for drug discovery that have not been exploited due to the lack of a suitable assay amenable to high throughput screening.

In both the translocase and transferase reactions a sugar molecule is transferred, from a nucleotide-linked precursor, to a lipid substrate. A conventional enzyme assay for both the translocase and the transferase involves using a radiolabelled sugar precursor and monitoring incorporation of the radiolabel into the lipid product. The lipid product is monitored either by paper chromatography or by extraction of the product in butanol: 6M pyridinium acetate, pH4.1 (2:1 v/v). In the paper chromatogram both the lipid products Lipid I and Lipid II run with an Rf of ~0.9.

Another known assay which monitors only translocase activity uses a dansylated UDP-MurNAc-pentapeptide as a substrate which is fluorescent. When the fluorescent substrate is transferred to the lipid carrier in the membrane, it undergoes a change in its environment from an aqueous to a hydrophobic one. This causes a blue shift in its emission spectrum (525 nm to 495 nm) which is monitored during the assay. Change in the intensity of fluorescence is only two- to three-fold and therefore it is not a very sensitive assay.

A high throughput radioactive assay for the transferase enzyme has been described in WO 99/38958 but this requires chemical synthesis of an artificial substrate.

It would be desirable to develop a method for assaying the activity of the translocase enzyme and/or transferase enzyme which is suitable for high throughput screening.

In accordance with the present invention, there is therefore provided a method for assaying UDP-*N*-acetylglucosamine: *N*-acetylmuramyl(pentapeptide)-P-P-undecaprenol-*N-*acetylglucosamine transferase enzyme activity, and, optionally also phospho-*N*-acetylmuramyl-pentapeptide translocase enzyme activity, which method comprises the steps of:
(1) incubating a reaction mixture comprising undecaprenol-pyrophosphate-*N-*acetylmuramylpentapeptide (Lipid I), radiolabelled UDP-*N*-acetyl glucosamine (UDP-Glc*N*Ac), a source of divalent metal ions and a source of the transferase enzyme and wherein bacterial cell membranes represent a source of one or more of undecaprenyl phosphate, translocase enzyme and transferase enzyme and wherein the bacterial cell membranes are obtained from a mutant deficient in peptidoglycan transglycosylase enzyme under conditions suitable for synthesis of undecaprenol-pyrophosphoryl-*N-*acetylmuramylpentapeptide-*N*-acetylglucosamine (Lipid II) to occur;
(2) stopping the reaction of step (1);
(3) adding to the reaction mixture of step (2) a fluorescer; and
(4) measuring light energy emitted by the fluorescer.

In the context of the present specification, it should be understood that the abbreviation "UDP" refers to uriditte (5'-)diphosphate.

The method according to the present invention is very conveniently carried out using 96-well microtitre plates, thereby enabling a fast, simple and reproducible way of measuring enzyme activity.

If it is intended to assay both the transferase and translocase enzymes, then in step (1), the Lipid I is formed *in situ* by including in the reaction mixture UDP-*N-*acetylmuramylpentapeptide (UDP-Mur*N*Ac-pentapeptide), a source of undecaprenyl phosphate and a source of the translocase enzyme.

The UDP-Mur*N*Ac-pentapeptide used may be any of those usually present in naturally-occurring peptidoglycans and is conveniently purified from bacteria or made enzymatically with precursors from bacteria, e.g. by methods similar to that described by T. den Blaauwen, M. Aarsman and N. Nanninga, J. Bacteriol., (1990), **172**, 63-70). A preferred UDP-Mur*N*Ac-pentapeptide to use is UDP-Mur*N*Ac-L-alanine-γ-D-glutamic acid-m-diaminopimelic acid-D-alanine-D-alanine from *Bacillus cereus.* The concentration of UDP-Mur*N*Ac-pentapeptide used will typically be in the range from 5 µM to 300µM, preferably from 5 µM to 200µM, more preferably from 5 µM to 100µM, and especially from 5 µM to 50µM, particularly 15 µM, per well of the microtitre plate.

As radiolabelled UDP-*N*-acetyl glucosamine, it is convenient to use tritiated UDP-*N*-acetyl glucosamine (UDP-[³H]Glc*N*Ac, commercially available from NEN-Dupont), preferably in a concentration of from 0.25 to 25µM per well of the microtitre plate, e.g. at a concentration of 2.5µM with 0.1 to 0.5 µCi radioactivity per well, preferably 0.2 µCi per well of the microtitre plate.

The divalent metal ions used are preferably magnesium ions. A suitable source of magnesium ions is magnesium chloride, preferably at a concentration in the range from 10 to 30mM, preferably from 10 to 25mM.

The membranes of *Escherichia coli* bacteria may conveniently be used and indeed are preferred as a source of undecaprenyl phosphate, translocase enzyme and transferase enzyme. The quantity of membranes used will typically be in the range from 1 to 20µg, particularly from 4 to 6µg, protein per well of the microtitre plate. The membranes may be prepared as described in Example 1 of WO 99/60155. Since the method according to the present invention monitors the amount of radiolabel incorporated into Lipid II, it is important when using a membrane preparation to ensure that the transglycosylase enzyme present is made ineffective, so that the radiolabelled disaccharide from Lipid II is not transferred to peptidoglycan also present in the membrane preparation by the activity of the transglycosylase enzyme. This is achieved by using membranes from e.g. an *Escherichia coli* mutant that are defective for the transglycosylase enzyme (for example, as described in WO 96/16082).

In step (1), it may be convenient to use an aqueous medium such as a buffer solution, e.g. of HEPES-ammonia, HEPES- KOH (HEPES being N-[2-Hydroxyethyl]piperazine-N'-[2-ethanesulfonic acid]) or Tris[hydroxymethyl]aminomethane hydrochloride ("Tris-HCl"), the buffer solution having a pH of about 7.5. HEPES and Tris-HCl are commercially available from the Sigma-Aldrich Co. Ltd.

The reaction mixture of step (1) is maintained at a temperature in the range from 20°C to 37°C for a period of 2 to 90 minutes, e.g. 5 minutes, under conditions suitable for enzyme-catalysed Lipid II synthesis to occur.

If the method according to the invention is intended to be used as a screen for identifying anti-bacterial compounds that are antagonists of the transferase enzyme and optionally the translocase enzyme, the reaction mixture of step (1) may further comprise one or more test compounds in varying concentrations. Since the translocase and transferase enzymes are essential for bacterial growth and are located on the cell surface, these enzymes represent good targets for the development of anti-bacterial drugs. Any such drugs would have the advantage that they would not need to enter the bacterial organism through the cell wall to be effective and thus the usual difficulties of cell wall permeability and drug resistance brought about by changes in cell wall permeability and efflux would be avoided.

The reaction is stopped (or quenched) in step (2) by any suitable means, for example, by adding a quenching agent. If the transferase enzyme is being assayed alone, then further reaction is conveniently stopped by adding an excess of unlabelled UDP-*N*-acetyl glucosamine. Alternatively, if the transferase and translocase enzymes are being assayed together, then further reaction may be stopped by adding a suitable amount of a divalent metal ion chelator compound, e.g. ethylenediaminetetraacetic acid (EDTA) which is commercially available from the Sigma-Aldrich Co. Ltd. The concentration of the chelator compound will of course depend on the particular chelator compound used and should be sufficient to chelate all the divalent metal ions; in the case of EDTA the concentration will typically be about 15 mM per well of the microtitre plate.

In step (3), the fluorescer used may be any of those routinely employed in scintillation proximity assays. The fluorescer will usually be associated with or supported by, in or on beads, for example, lectin-coated beads, anti-mouse antibody coated yttrium silicate beads, polylysine (e.g. poly(L)lysine)-coated yttrium silicate beads, Protein A-coated yttrium silicate beads, anti-mouse antibody coated PVT (polyvinyltoluene) beads or wheatgerm agglutinin-coated PVT beads, all of which beads are commercially available from Amersham Inc. The beads chosen should be capable of binding to bacterial cell walls.

It is preferred to use lectin-coated beads impregnated with a fluorescer, for example, as described in US Patent No. 4,568,649 and European Patent No. 154,734. The beads (known as "Scintillation Proximity Assay" (or SPA) beads) are commercially available from Amersham Inc. Most preferred are wheatgerm agglutinin-coated SPA beads which are capable of binding sugar molecules, specifically N-acetyl glucosamine. It is believed that through the binding of N-acetyl glucosamine to the SPA beads, radiolabelled Lipid II formed in step (1) is brought into close proximity with the fluorescer which becomes activated by the radiation energy, resulting in the emission of light energy which is subsequently measured in step (4).

The beads (with fluorescer), which are conveniently added in the form of an aqueous suspension, are contacted with the reaction mixture of step (2) for a period of at least 10 minutes, preferably 3 hours or more (e.g. overnight), before the plate is "counted" in step (4), e.g., in a "Microbeta Tilux" counter.

The present invention also provides a method for assaying phospho-*N*-acetylmuramyl-pentapeptide translocase enzyme activity, which method comprises the steps of:
(A) incubating a reaction mixture comprising a UDP-*N*-acetylmuramylpentapeptide (UDP-Mur*N*Ac-pentapeptide), a radiolabelled derivative of a UDP-*N-*acetylmuramylpentapeptide, a source of divalent metal ions, a source of undecaprenyl phosphate and a source of the translocase enzyme under conditions suitable for the formation of a coupled product between the radiolabelled derivative and the undecaprenyl phosphate;
(B) stopping the reaction of step (A);
(C) adding to the reaction mixture of step (B) a fluorescer; and
(D) measuring light energy emitted by the fluorescer.

In step (A), the UDP-Mur*N*Ac-pentapeptide used may be any of those usually present in naturally-occurring peptidoglycans and is conveniently purified from bacteria or made enzymatically with precursors from bacteria, e.g. by methods similar to that described by T. den Blaauwen, M. Aarsman and N. Nanninga, J. Bacteriol., (1990), **172**, 63-70). A preferred UDP-Mur*N*Ac-pentapeptide to use is UDP-Mur*N*Ac-L-alanine-γ-D-glutamic acid-m-diaminopimelic acid-D-alanine-D-alanine from *Bacillus cereus.*

The radiolabelled derivative of a UDP-*N*-acetylmuramylpentapeptide preferably contains tritium [³H], ³³P or ¹²⁵I. Such a compound may be synthesized, for example, by incorporating ³H-propionate at the ε-amino group of the meso-DAP residue of UDP-Mur*N*Ac-L-alanine-γ-D-glutamic acid-m-diaminopimelic acid-D-alanine-D-alanine.

The total amount of UDP-Mur*N*Ac-pentapeptide and of radiolabelled derivative will typically be in the range from 4 µM to 15µM, preferably from 4 µM to 10µM, e.g. from 4.5 µM to 5.5µM, per well of the microtitre plate. The amount of the radiolabelled derivative used is such that the radioactivity measures from, e.g., 0.1 µCi to 0.6 µCi per well, preferably from 0.1 µCi to 0.4 µCi per well, particularly 0.2 µCi per well.

The divalent metal ions used are the same as those previously described.

The membranes of *Escherichia coli* bacteria may conveniently be used and indeed are preferred as a source of undecaprenyl phosphate and translocase enzyme. The quantity of membranes used will typically be in the range from 5 to 25µg, particularly from 10 to 15µg, protein per well of the microtitre plate. The membranes may be prepared as described in Example 1 of WO 99/60155.

In step (A), it may be convenient to use an aqueous medium such as a buffer solution, e.g. of HEPES-ammonia, HEPES- KOH (HEPES being N-[2-Hydroxyethyl]piperazine-N'-[2-ethanesulfonic acid]) or Tris[hydroxymethyl]aminomethane hydrochloride ("Tris-HCl"), the buffer solution having a pH of about 7.5. HEPES and Tris-HCl are commercially available from the Sigma-Aldrich Co. Ltd.

The reaction mixture of step (A) is maintained at a temperature in the range from 20°C to 37°C for a period of 2 to 15 minutes, e.g. 8 minutes, under conditions suitable for enzyme-catalysed Lipid I synthesis to occur.

In a preferred aspect, the reaction mixture of step (A) will additionally comprise suitable amounts of detergent (such as Triton X-100 at 0.1%w/v) and potassium chloride, to improve the signal observed when carrying out step (D) of the method of the invention.

If the method according to the invention is intended to be used as a screen for identifying anti-bacterial compounds that are antagonists of the translocase enzyme, the reaction mixture of step (A) may further comprise one or more test compounds in varying concentrations.

The reaction is stopped (or quenched) in step (B) by any suitable means, for example, by the addition, as quenching agent, of a suitable amount of a divalent metal ion chelator compound, e.g. ethylenediaminetetraacetic acid (EDTA) which is commercially available from the Sigma-Aldrich Co. Ltd. The concentration of the chelator compound will of course depend on the particular chelator compound used and should be sufficient to chelate all the divalent metal ions; in the case of EDTA the concentration will typically be about 35 mM per well of the microtitre plate.

In step (C), the fluorescer used may be any of those routinely employed in scintillation proximity assays. The fluorescer will usually be associated with or supported by, in or on beads, for example, lectin-coated beads, anti-mouse antibody coated yttrium silicate beads, polylysine (e.g. poly(L)lysine)-coated yttrium silicate beads, Protein A-coated yttrium silicate beads, anti-mouse antibody coated PVT (polyvinyltoluene) beads or wheatgerm agglutinin-coated PVT beads, all of which beads are commercially available from Amersham Inc. The beads chosen should be capable of binding to bacterial cell walls.

It is preferred to use lectin-coated beads impregnated with a fluorescer, for example, as described in US Patent No. 4,568,649 and European Patent No. 154,734. The beads (known as "Scintillation Proximity Assay" (or SPA) beads) are commercially available from Amersham Inc. Most preferred are wheatgerm agglutinin-coated SPA beads which are capable of binding sugar molecules, specifically N-acetyl glucosamine. It is believed that the coupled product is captured onto the lectin-coated beads through the binding of N-acetyl glucosamine which is present in the cell wall fragments associated with the bacterial membranes if these are used in the method of the invention. Due to specific capture of the coupled product, the radiolabel is brought into close proximity with the fluorescer which becomes activated by the radiation energy, resulting in the emission of light energy which is subsequently measured in step (D).

The beads (with fluorescer) which are conveniently added in the form of an aqueous suspension are contacted with the reaction mixture of step (B) for a period of at least 10 minutes, preferably 3 hours or more (e.g. overnight), before the plate is "counted" in step (D), e.g., in a "Microbeta Tilux" counter.

The present invention will be further explained by reference to the following illustrative examples.

### Example 1

(i) The wells of a microtitre plate were individually filled with a total volume of 25 µl of a reaction mixture comprising an aqueous buffer solution of 50 mM HEPES-ammonia (N-[2-Hydroxyethyl]piperazine-N'-[2-ethanesulfonic acid]) (pH 7.5), and 10 mM magnesium chloride, 15 µM UDP-Mur*N*Ac-L-alanine-γ-D-glutamic acid-m-diaminopimelic acid-D-alanine-D-alanine, 2.5 µM tritiated UDP-*N*-acetyl glucosamine (0.2 µCi per well), 3 µM Moenomycin, 4 µg of *Escherichia coli* AMA1004 cell membranes and a solution of test compound (e.g. Tunicamycin, Vancomycin, Nisin) of varying concentration in 4% dimethylsulphoxide. Tunicamycin is a known antagonist of the translocase enzyme, Nisin is a known antagonist of the transferase enzyme and Vancomycin is a known antagonist of both the translocase and transferase enzymes. (Moenomycin is a known antagonist of the transglycosylase enzyme and is added to prevent the radiolabel from being incorporated into peptidoglycan).

Four wells of the microtitre plate were used as controls: two wells contained no UDP-*N*-acetylmuramylpentapeptide (0% reaction controls) and a further two wells contained no test compound (100% reaction controls).

If the purpose of the screen is to study the effect of an inhibitor of the transferase or translocase, the test compound is added along with the substrates at step (i).

The E. coli membranes were prepared as described in patent application WO 99/60155.

The microtitre plate was incubated at 37 °C for 5 min and thereafter 5 µl of ethylenediaminetetraacetic acid (EDTA) was added to give a final EDTA concentration of 15 mM.
(ii) After addition of the EDTA, 170 µl of an aqueous suspension of wheatgerm agglutinin-coated scintillation proximity assay beads comprising 500 µg beads in a solution of HEPES-ammonia, pH 7.5, was added to each well such that the final concentration of HEPES-ammonia was 50 mM.

The plate was left for 3 hours/overnight at room temperature before being counted in the "Microbeta Trilux" counter.

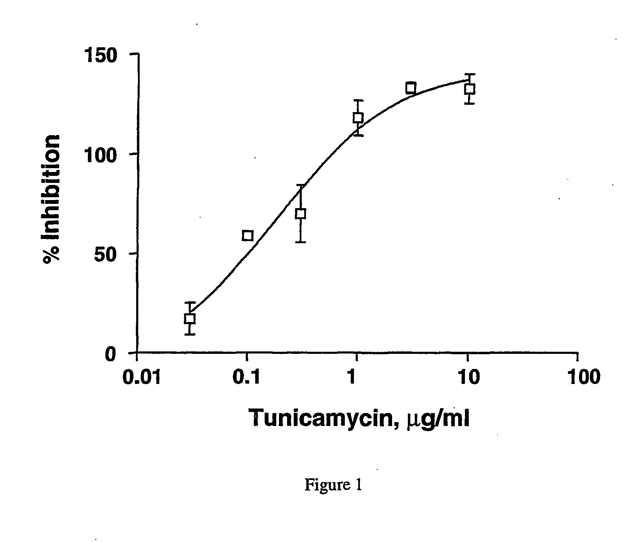

Figure 1 is a graph showing the percentage inhibition of translocase (and thus Lipid II synthesis) versus Tunicamycin concentration (after subtracting the corresponding 0% reaction readings).

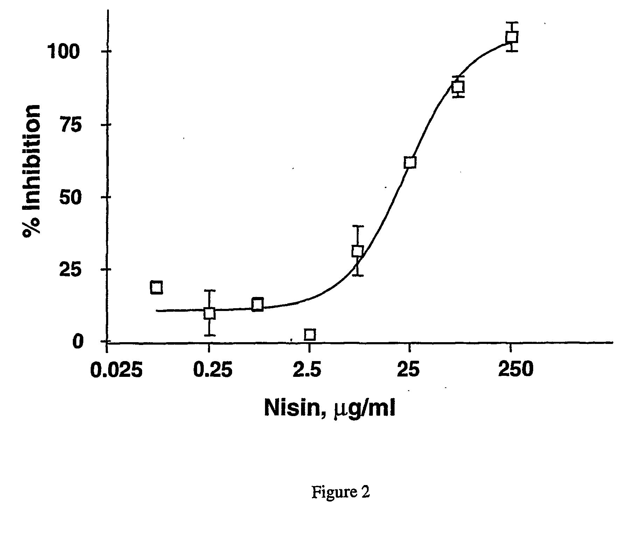

Figure 2 is a graph showing the percentage inhibition of transferase (and thus Lipid II synthesis) versus Nisin concentration (after subtracting the corresponding 0% reaction readings).

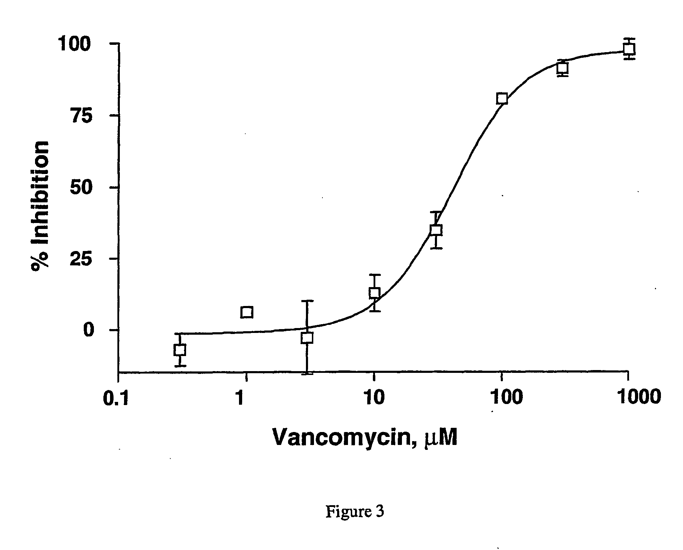

Figure 3 is a graph showing the percentage inhibition of translocase and transferase (and thus Lipid II synthesis) versus Vancomycin concentration (after subtracting the corresponding 0% reaction readings).

### Example 2

The method described in Example 1 may alternatively be performed using the membranes of an *Escherichia coli* mutant, AMA1004 Δpon B :: Spc^{R}, a mutant from which the gene ponB encoding PBPlb has been inactivated, as described by S.Y. Yousif, J.K. Broome-Smith and B.G. Spratt, *J. Gen. Microbiol*., (1985), **131,** 2839-2845. These membranes lack PBP1b activity which is the major transglycosylase in *Escherichia coli* and thus the radiolabel incorporated into Lipid II is not transferred to peptidoglycan. Hence there is no need to add moenomycin to the reaction mixture.

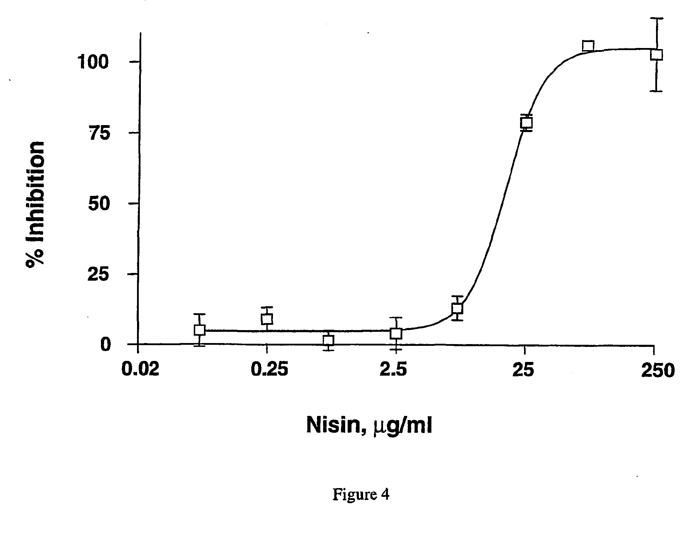

Figure 4 is a graph showing the percentage inhibition of transferase (and thus Lipid II synthesis) versus Nisin concentration (after subtracting the corresponding 0% reaction readings).

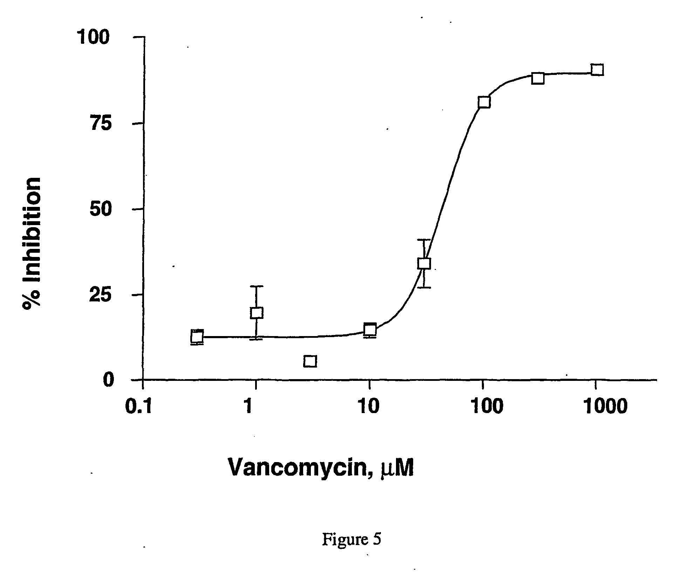

Figure 5 is a graph showing the percentage inhibition of translocase and transferase (and thus Lipid II synthesis) versus Vancomycin concentration (after subtracting the corresponding 0% reaction readings).

### Example 3

(i) The wells of a microtitre plate were individually filled with a total volume of 25 µl of a reaction mixture comprising an aqueous buffer solution of 50 mM HEPES-ammonia (N-[2-Hydroxyethyl]piperazine-N'-[2-ethanesulfonic acid]) (pH 7.5), and 10 mM magnesium chloride 15 µM UDP-Mur*N*Ac-L-alanine-γ-D-glutamic acid-m-diaminopimelic acid-D-alanine-D-alanine, 2.5 µM tritiated UDP-*N*-acetyl glucosamine (0.2 µCi per well), 6 µg of *Escherichia coli* AMA1004 cell membranes prepared as described below and a solution of test compound (e.g. Tunicamycin, Vancomycin) of varying concentration in 4% dimethylsulphoxide. Tunicamycin is a known antagonist of the translocase enzyme and Vancomycin is a known antagonist of both the translocase and transferase enzymes.

Four wells of the microtitre plate were used as controls: two wells contained no UDP-*N*-acetylmuramylpentapeptide (0% reaction controls) and a further two wells contained no test compound (100% reaction controls).

If the purpose of the screen is to study the effect of an inhibitor of the transferase or translocase the test compound is added along with the substrates at step (i).

The E. coli membranes were prepared as follows.
Four to five colonies of the bacteria from an LB (Luria Bertani medium) agar plate were inoculated into 5 ml LB-broth and grown during the day (for 6-8 hours) at 37°C. In the evening 0.5 ml of this culture was used to inoculate 500 ml of LB-broth in a 2 1 flask. The flask was incubated on a shaker at 30°C overnight; typically an A600 of 2.0-2.5 was reached. Early the next morning this culture was used to inoculate 6 1 of LB-broth (using 500 ml of LB-broth per 21 flask) such that the starting A600 was 0.4-0.6. The culture was grown for 2 hours at 37°C with vigorous shaking/aeration; the A600 reached was between 1.4 and 2.0. At this point the bacteria were cooled on ice and pelleted by centrifugation at 5,000 x g for 15 minutes. The cell pellet was washed with 500 ml of Buffer A (50 mM Tris-HCl, pH 7.5 / 0.1 mM MgCl₂). They were resuspended in cold 20% sucrose in 20 mMTris-HCl pH8.0 with (a volume that is 7.5 times the wet weight of cells). Lysozyme was added to a concentration of 200 ug/ml and the cells gently stirred for 10 min on ice. A solution of EDTA was added, over a 1 hour period, to a final concentration of 0.02 M. The cells were spun at 12,000 x g for 20 min and the pellet obtained from this spin was resuspended in 50 mM Tris-HCl, pH 7.5, containing 1 mM MgCl₂ and RNase and DNase to a final concentration of 20µg/ml each. The suspension was gently stirred for 1 hr at room temperature. The cell lysate was spun at 3,500 x g for 45 minutes. The supernatant was collected, diluted to 100 ml with Buffer A and ultra-centrifuged at 150,000 x g for 45 minutes. The pellet from this spin was washed by resuspending it in 100 ml of Buffer A and re-centrifuging at 150,000 x g for 30 minutes. This pellet was gently resuspended in a minimal volume (5-10 ml for 61 culture) of Buffer A and frozen and stored in aliquots at -70°C. This is termed the membrane preparation and was used in the assay as a source of the translocase and transferase enzymes and undecaprenyl phosphate.

The microtitre plate was incubated at 37 °C for 30 min and thereafter 5 µl of ethylenediaminetetraacetic acid (EDTA) was added to give a final EDTA concentration of 15 mM.
(ii) After addition of the EDTA, 170 µl of an aqueous suspension of wheatgerm agglutinin-coated scintillation proximity assay beads comprising 500 µg beads in a solution of HEPES-ammonia, pH 7.5, was added to each well such that the final concentration of HEPES-ammonia was 50 mM.

The plate was left for 3 hours/ overnight at room temperature before being counted in the "Microbeta Trilux" counter.

Table 1 below enumerates the inhibitory effects of Tunicamycin and Vancomycin on the translocase and transferase enzymes (after subtracting the corresponding 0% reaction readings).

**Table 1**

| **Test Compound** | **Concentration** | **% Inhibition** |
|---|---|---|
| Tunicamycin | 6µg/ml | 104 |
| Vancomycin | 100µM | 82 |

### Example 4

(i) The wells of a microtitre plate were individually filled with a total volume of 15 µl of a reaction mixture comprising an aqueous buffer solution of 50 mM HEPES-ammonia (pH 7.5) (N-[2-Hydroxyethyl]piperazine-N'-[2-ethanesulfonic acid]) and 10 mM magnesium chloride, 15 µM UDP-Mur*N*Ac-L-alanine-γ-D-glutamic acid-m-diaminopimelic acid-D-alanine-D-alanine, and 4 µg of the cell membranes of the *Escherichia coli* mutant, AMA1004 Δpon B :: Spc^{R}, a mutant from which the gene ponB encoding PBPlb has been inactivated, as described by S.Y. Yousif, J.K. Broome-Smith and B.G. Spratt, *J. Gen. Microbiol.,* (1985), **131,** 2839-2845. The plate was incubated for 20 min at 37°C.
(ii) Tunicamycin was then added to a final concentration of 10µg/ml, followed by a test compound (e.g. Vancomycin or Nisin) of varying concentration in dimethyl sulphoxide. Nisin and Vancomycin are known antagonists of the transferase enzyme.
(iii) Then to the reaction well, in a 5 µl volume, the substrate for the transferase was added: 2.5 µM tritiated UDP-*N*-acetyl glucosamine (0.5 µCi per well). The microtitre plate was incubated for 5 min at 37°C.
(iv) The reaction was terminated by diluting out the radiolabel i.e. by addition of 25µl of 200 µM unlabelled UDP-GlcNAc.
(v) After addition of the UDP-GlcNAc, 150 µl of an aqueous suspension of wheatgerm agglutinin-coated scintillation proximity assay beads comprising 500 µg beads in a solution of HEPES-ammonia, pH 7.5, were added to each well such that the final concentration of HEPES-ammonia was 50 mM. The plate was left for 3 hours at room temperature before being counted in the "Microbeta Trilux" counter.

Four wells of the microtitre plate were used as controls: two wells contained no UDP-*N*-acetylmuramylpentapeptide (0% reaction controls) and a further two wells contained no test compound (100% reaction controls).

Table 2 below enumerates the inhibitory effects of Nisin and Vancomycin on the transferase enzyme (after subtracting the corresponding 0% reaction readings).

**Table 1**

| **Test Compound** | **Concentration** | **% Enzyme Activity** |
|---|---|---|
| Nisin | 10µg/ml | ~30 |
| Vancomycin | 100µM | ~50 |

### Example 5

(i) The wells of a microtitre plate were individually filled with a total volume of 25 µl of a reaction mixture comprising an aqueous buffer solution of 100 mM HEPES ammonia pH 7.5, 25 mM magnesium chloride , 50 mM KCl, 0.1% w/v Triton X-100, 4 µM UDP-MurNAc-pentapeptide plus UDP-MurNAc-[³H]-pentapeptide (0.2 µCi per well), 12.5 µg of the cell membranes of the *Escherichia coli* mutant, AMA1004 Δpon B :: Spc^{R} (a mutant from which the gene ponB encoding PBP1b has been inactivated, as described by S.Y. Yousif, J.K. Broome-Smith and B.G. Spratt, *J. Gen. Microbiol*., (1985), 131, 2839-2845) and a solution of test compound (e.g. Tunicamycin, Vancomycin) of varying concentration. Tunicamycin and Vancomycin are known antagonists of the translocase enzyme.

The *E. coli* membranes were prepared as described in patent application WO 99/60155.

UDP-Mur*N*Ac-[³H]-pentapeptide was synthesised as follows.
20 nanomoles of UDP-Mur*N*AC-pentapeptide (purified from the hot water extracts of *B.cereus)* were incubated with 1 mCi of ³H-N-hydroxy succinimidyl propionate (specific activity - 91 Ci/mmol) in 20 µl of 100 mM sodium borate buffer, pH 8.5 at 4°C for 20 hrs. Reaction mix was diluted to 100 µl total volume using 80 µl of 0.1 M ammonium acetate buffer, pH 8.5, and loaded on 500 µl DEAE sepharose column equilibrated in the same buffer. Column was washed with six to seven ml of 0.1 M ammonium acetate buffer, pH 8.5, to remove the unbound, unreacted ³H-NHS-propionate. The bound product, UDP-Mur*N*Ac-L-Ala-γ-D-Glu-m-DAP(N^{ε}-³H-Propionate)-D-Ala-D-Ala was eluted using 0.5 M ammonium acetate buffer, pH 8.5. Fractions, 0.5 ml each, were collected and monitored for activity by using them as a substrate in the enzyme assay. Active fractions were pooled and the specific activity was determined.

Four wells of the microtitre plate were used as controls: two wells contained stop solution at zero time point (0% reaction controls) and a further two wells contained no test compound (100% reaction controls).
(ii) The microtitre plate was incubated at 22°C for 8 minutes.
(iii) EDTA (5 µl) was added to a final concentration of 35 mM and thereafter 270 µl of an aqueous suspension of wheatgerm agglutinin-coated scintillation proximity assay beads comprising 2000 µg beads in a solution of HEPES ammonia, pH 7.5, and sodium azide was added to each well to reach the final concentration of 100 mM HEPES and 0.02 % w/v sodium azide respectively.

The plate was left for 15 hours at room temperature before being counted in the "Microbeta Trilux" counter.

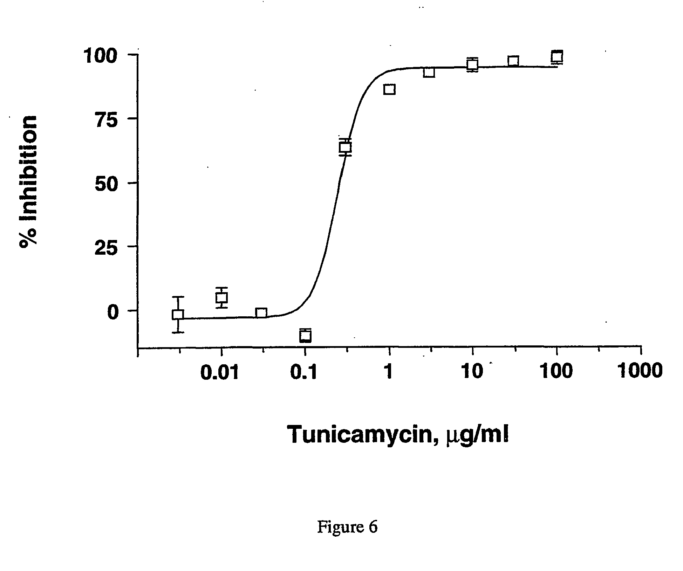

Figure 6 is a graph showing the percentage inhibition of translocase (and thus Lipid I synthesis) versus Tunicamycin concentration (after subtracting the corresponding 0% reaction readings).

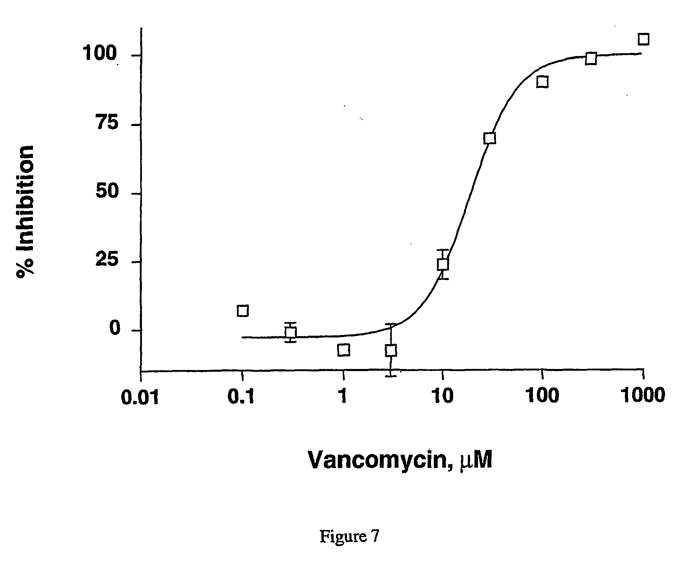

Figure 7 is a graph showing the percentage inhibition of translocase (and thus Lipid I synthesis) versus Vancomycin concentration (after subtracting the corresponding 0% reaction readings).

## Claims

1. A method for assaying UDP-*N*-acetylglucosamine: *N*-acetylmuramyl(pentapeptide)-P-P-undecaprenol-*N*-acetylglucosamine transferase enzyme activity, and, optionally also phospho-*N*-acetylmuramyl-pentapeptide translocase enzyme activity,
which method comprises the steps of:
(1) incubating a reaction mixture comprising undecaprenol-pyrophosphate-*N-*acetylmuramylpentapeptide (Lipid I), radiolabelled UDP-*N*-acetylglucosamine (UPP-Glc*N*Ac), a source of divalent metal ions and a source of the transferase enzyme and wherein bacterial cell membranes represent a source of one or more of undecaprenyl phosphate, translocase enzyme and transferase enzyme and wherein the bacterial cell membranes are obtained from a mutant deficient in peptidoglycan transglycosylase enzyme under conditions suitable for synthesis of undecaprenol-pyrophosphoryl-*N-*acetylmuramylpentapeptide-*N*-acetylglucosamine (Lipid II) to occur;
(2) stopping the reaction of step (1);
(3) adding to the reaction mixture of step (2) a fluorescer; and
(4) measuring light energy emitted by the fluorescer.

2. A method according to claim 1, wherein the Lipid I is formed *in situ* from an UDP-*N-*acetylmuramylpentapeptide and a source of undecaprenyl phosphate, in the presence of a source of phospho-*N*-acetylmuramyl-pentapeptide translocase enzyme.

3. A method according to claim 2, wherein the UDP-*N*-acetylmuramylpentapeptide is UDP-Mur*N*Ac-L-alanine-γ-D-glutamic acid-m-diaminopimelic acid-D-alanine-D-alanme and wherein bacterial cell membranes represent a source of one or more of undecaprenyl phosphate, translocase enzyme and transferase enzyme and the reaction mixture of step (1) optionally further comprises a peptidoglycan transglycosylase enzyme inhibitor.

4. A method according to claim 1, wherein the bacterial cell membranes are from *Escherichia coli*.

5. A method according to claim 1, wherein the peptidoglycan transglycosylase enzyme inhibitor is moenomycin and wherein the bacterial cell membranes are obtained from a mutant deficient in peptidoglycantransglycosylase enzyme.

6. A method according to any one of claims 1 to 5, wherein magnesium chloride is used as a source of divalent metal ions.

7. A method according to any one of claims 1 to 6, wherein the reaction mixture of step (1) further comprises a test compound.

8. A method according to claim 7, wherein the test compound is an antagonist of the translocase enzyme or the transferase enzyme.

9. A method according to any one of claims 1 to 8, wherein in step (2) an excess of unlabelled UDP-*N*-acetyl glucosamine or a divalent metal ion chelator compound is added.

10. A method according to any one of claims 1 to 9, wherein the fluorescer is associated with or supported by, in or on lectin-coated beads, anti-mouse antibody coated beads or polylysine coated beads.

## Patentansprüche

1. Verfahren zum Testen der Enzymaktivität von UDP-*N-*acetylglucosamin:*N*-Acetylmuramyl(pentapeptid)-P-P-undecaprenol-N-acetylglucosamin-Transferase und gegebenenfalls ebenso der Enzymaktivität von Phospho-N-acetylmuramylpentapeptid-Translokase,
wobei man die folgenden Schritte ausführt:
(1) Inkubieren eines Reaktionsansatzes, umfassend Undecaprenolpyrophosphat-*N-*acetylmuramylpentapeptid (Lipid I), radioaktiv markiertes UDP-N-acetylglucosamin (UDP-Glc*N*Ac), eine Quelle für zweiwertige Metallionen sowie eine Quelle für das Transferaseenzym, und wobei Bakterienzellmembranen eine Quelle für ein oder mehrere Mitglieder der Gruppe Undecaprenylphosphat, Translokaseenzym und Transferaseenzym darstellen und wobei die Bakterienzellmembranen aus einer Peptidoglycan-Transglycosylaseenzym-Mangelmutante gewonnen werden, unter für den Ablauf der Synthese von Undecaprenolpyrophosphoryl-*N*-acetylmuramylpentapeptid-*N*-acetylglucosamin (Lipid II) geeigneten Bedingungen;
(2) Abstoppen der Reaktion aus Schritt (1);
(3) Versetzen des Reaktionsansatzes aus Schritt (2) mit einer Fluoreszenzverbindung; und
(4) Messen der von der Fluoreszenzverbindung emittierten Lichtenergie.

2. Verfahren nach Anspruch 1, wobei das Lipid I in situ aus einem UDP-N-acetylmuramylpentapeptid und einer Undecaprenylphosphat-Quelle in Gegenwart einer Phospho-N-acetylmuramylpentapeptid-Translokaseenzym-Quelle gebildet wird.

3. Verfahren nach Anspruch 2, wobei es sich bei dem UDP-N-acetylmuramylpentapeptid um UDP-MurNAc-L-alanin-γ-D-glutaminsäure-m-diaminopimelinsäure-D-alanin-D-alanin handelt und wobei Bakterienzellmembranen eine Quelle für ein oder mehrere Mitglieder der Gruppe Undecaprenylphosphat, Translokaseenzym und Transferaseenzym darstellen und der Reaktionsansatz aus Schritt (1) gegebenenfalls ferner einen Peptidoglycan-Transglycosylaseenzym-Inhibitor umfaßt.

4. Verfahren nach Anspruch 1, wobei die Bakterienzellmembranen aus *Escherichia coli* stammen.

5. Verfahren nach Anspruch 1, wobei es sich bei dem Peptidoglycan-Transglycosylaseenzym-Inhibitor um Moenomycin handelt und wobei die Bakterienzellmembranen aus einer Peptidoglycan-Transglycosylaseenzym-Mangelmutante gewonnen werden.

6. Verfahren nach einem der Ansprüche 1 bis 5, wobei als Quelle für zweiwertige Metallionen Magnesiumchlorid eingesetzt wird.

7. Verfahren nach einem der Ansprüche 1 bis 6, wobei der Reaktionsansatz aus Schritt (1) ferner eine Testverbindung umfaßt.

8. Verfahren nach Anspruch 7, wobei es sich bei der Testverbindung um einen Antagonisten des Translokaseenzyms oder des Transferaseenzyms handelt.

9. Verfahren nach einem der Ansprüche 1 bis 8, wobei in Schritt (2) ein Überschuß an nicht markiertem UDP-N-acetylglucosamin oder eine Chelatorverbindung für zweiwertige Metallionen zugegeben wird.

10. Verfahren nach einem der Ansprüche 1 bis 9, wobei die Fluoreszenzverbindung mit mit Lectin beschichteten Kügelchen, mit Anti-Maus-Antikörper beschichteten Kügelchen oder mit Polylysin beschichteten Kügelchen assoziiert oder davon, darin oder darauf geträgert ist.

## Revendications

1. Procédé pour tester l'activité enzymatique de l'UDP-N-acétylglucosamine:N-acétylmuramyl(pentapeptide)-P-P-undécaprénol-N-acétylglucosamine transférase et éventuellement aussi l'activité enzymatique de la phospho-N-acétylmuramyl-pentapeptide translocase, ledit procédé comprenant les étapes:
(1) d'incubation d'un mélange réactionnel comprenant de l'undécaprénol-pyrophosphate-N-acétylmuramyl-pentapeptide (lipide I), de l'UDP-N-acétylglucosamine radiomarquée (UDP-GlcNAc), une source d'ions métalliques divalents et une source de l'enzyme transférase et où les membranes de cellules bactériennes représentent une source d'un ou de plusieurs parmi l'undécaprénylphosphate, l'enzyme translocase et l'enzyme transférase et où les membranes de cellules bactériennes sont obtenues à partir d'un mutant déficient en enzyme peptidoglycan transglycosylase dans des conditions appropriées pour que la synthèse de l'undécaprénol-pyrophosphoryl-N acétylmuramylpentapeptide-N-acétylglucosamine (lipide II) se produise;
(2) d'arrêt de la réaction de l'étape (1);
(3) d'addition d'un agent de fluorescence au mélange réactionnel de l'étape (2) et
(4) de mesure de l'énergie lumineuse émise par l'agent de fluorescence.

2. Procédé selon la revendication 1, dans lequel le lipide I est formé in situ à partir d'un UDP-N-acétylmuramylpentapeptide et d'une source de undécaprénylphosphate, en présence d'une source de l'enzyme phospho-N-acétylmuramyl-pentapeptide translocase.

3. Procédé selon la revendication 2, dans lequel l'UDP-N-acétylmuramylpentapeptide est l'UDP-MurNAc-L-alanine-acide γ-D-glutamique-acide m-diaminopimélique-D-alanine-D-alanine et dans lequel des membranes de cellules bactériennes représentent une source d'un ou de plusieurs parmi l'undécaprénylphosphate, l'enzyme translocase et l'enzyme transférase et le mélange réactionnel de l'étape (1) comprend en outre éventuellement un inhibiteur de l'enzyme peptidoglycan transglycosylase.

4. Procédé selon la revendication 1, dans lequel les membranes de cellules bactériennes proviennent d'Escherichia coli.

5. Procédé selon la revendication 1, dans lequel l'inhibiteur de l'enzyme peptidoglycan transglycosylase est la moenomycine et dans lequel les membranes de cellules bactériennes sont obtenues à partir d'un mutant déficient en enzyme peptidoglycan transglycosylase.

6. Procédé selon l'une quelconque des revendications 1 à 5, dans lequel du chlorure de magnésium est utilisé comme source d'ions métalliques divalents.

7. Procédé selon l'une quelconque des revendications 1 à 6, dans lequel le mélange réactionnel de l'étape (1) contient en outre un composé test.

8. Procédé selon la revendication 7, dans lequel le composé test est un antagoniste de l'enzyme translocase ou de l'enzyme transférase.

9. Procédé selon l'une quelconque des revendications 1 à 8, dans lequel on ajoute dans l'étape (2) un excès d'UDP-N-acétyl-glucosamine non marqué ou un composé chélateur d'un ion métallique divalent.

10. Procédé selon l'une quelconque des revendications 1 à 9, dans lequel l'agent de fluorescence est associé à ou supporté par, dans ou sur des perles revêtues de lectine, des perles revêtues d'anticorps anti-souris ou revêtues de polylysine.
